# EUROPEAN PATENT APPLICATION

(11) **EP 4 108 236 A1**
(43) Date of publication of application: **28.12.2022**
(21) Application number: 21757123.1
(22) Date of filing: 20.02.2021
(51) Int. Cl.: A61K 9/48, A61K 9/20, A61K 9/14, A61K 31/502, A61K 47/40, A61P 35/00

(54) **OLAPARIB PHARMACEUTICAL COMPOSITION, PREPARATION THEREOF, PREPARATION METHOD THEREFOR AND USE THEREOF**

(30) Priority: 21.02.2020 CN 202010107404
(71) Applicant: Sinotherapeutics Inc., Shanghai 201210 (CN)
(72) Inventor: SHEN, Jianren, Shanghai 201210 (CN); LI, Zhao, Shanghai 201210 (CN); LI, Kun, Shanghai 201210 (CN); FANG, Larry Yun, Shanghai 201210 (CN); WAN, Jiansheng, Shanghai 201210 (CN)
(74) Representative: Reitstötter Kinzebach
(86) International application number: PCT/CN2021/077009
(87) International publication number: WO 2021/164755

(57) **Abstract**

A pharmaceutical composition, which contains Olaparib and the carrier material cyclodextrin, and can further contain another carrier material besides cyclodextrin and/or a solubilizer.

## Description

This application claims the priority of Chinese application No. 202010107404.6 filed on 21 February 2020, entitled "Olaparib pharmaceutical composition, formulation thereof, preparation method thereof and use thereof". The entire content of the Chinese application is incorporated herein by reference.

### Technical Field

The present disclosure relates to the field of pharmaceutical preparations, and particularly relates to a pharmaceutical composition comprising Olaparib and a formulation, a preparation process and use thereof.

### Background

Olaparib has the chemical name of 4-[(3-{[4(cyclopropylcarbonyl)piperazin-1-yl]carbonyl}-4-fluorophenyl)methyl]phthalazin-1(2H)-one, the molecular formula of C₂₄H₂₃FN₄O₃, the CAS number of 763113-22-0, and the structural formula as follows:

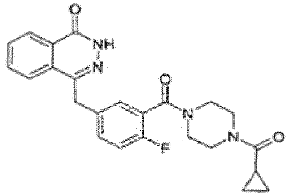

Olaparib is a poly(ADP-ribose) polymerase (PARR) inhibitor used in tumor therapy. It has been approved for marketing in the United States, Europe, Japan and China for the treatment of ovarian cancer, breast cancer and pancreatic cancer. In addition, relevant clinical trials for prostate cancer, non-small cell lung cancer, esophageal cancer, gastric cancer and other malignant tumors are underway.

CN102238945A discloses a solid dispersion containing Olaparib and copovidone VA 64, which is prepared by hot melt extrusion. CN104434809A uses povidone to replace copovidone as matrix polymer, and discloses a solid dispersion particle composed of Olaparib, povidone and lubricant. The hygroscopicity of the composition is smaller than that of the comparative example in which the copovidone is used as a carrier, and the preparation process is a hot melt extrusion. CN106692066A discloses a solid dispersion of Olaparib, povidone or copovidone, which is prepared by hot melt extrusion.

### SUMMARY

In one aspect, provided is a pharmaceutical composition comprising an active ingredient Olaparib and a carrier material cyclodextrin. In an embodiment, the cyclodextrin is one or more selected from the group consisting of α cyclodextrin, γ cyclodextrin, β cyclodextrin, hydroxypropyl β cyclodextrin, hydroxypropyl α cyclodextrin, sulfobutyl β cyclodextrin, and methyl β cyclodextrin, preferably hydroxypropyl β cyclodextrin.

The pharmaceutical composition of the present disclosure, in addition to the carrier material cyclodextrin (also referred to as "first carrier material"), may also contain a second carrier material.

Preferably, the second carrier material is one or more selected from the group consisting of copovidone, povidone, polyvinyl caprolactam-polyvinyl acetate-polyethylene glycol graft copolymer, polyoxyethylene, hypromellose and derivatives thereof, hydroxypropyl cellulose, methyl cellulose, hydroxyethyl cellulose, ethyl cellulose, sodium alginate, acrylic resin, and carbomer; more preferably, the second carrier material is one or more selected from the group consisting of copovidone, polyethylene glycol/vinyl caprolactam/vinyl acetate copolymer, acrylic resin, hypromellose and derivatives thereof; particularly preferably, the second carrier material is one or more selected from the group consisting of copovidone, and polyethylene glycol/vinyl caprolactam/vinyl acetate copolymer.

In a preferable embodiment, the pharmaceutical composition of the present disclosure may further comprise a solubilizer, such as polyethylene glycol 1000 vitamin E succinate (TPGS).

In another aspect, provided is a pharmaceutical formulation comprising the pharmaceutical composition of the present disclosure.

In yet another aspect, provided is use of the pharmaceutical composition or the pharmaceutical formulation of the present disclosure in the manufacture of a medicament for the treatment of a malignant tumors or related disease.

In another aspect, provided is a method for treating a malignant tumor or related disease, the method comprising administering to a subject in need thereof a pharmaceutical composition or pharmaceutical formulation of the present disclosure.

The pharmaceutical composition or pharmaceutical formulation of the present disclosure is for use in treating a malignant tumor or related disease.

The malignant tumor or related disease includes but is not limited to, an ovarian cancer, a breast cancer, a pancreatic cancer, a prostate cancer, a non-small cell lung cancer, an esophageal cancer or a gastric cancer.

### Brief Description of the Drawings

Figure 1 shows the Tg values corresponding to different Kleptose contents in pharmaceutical compositions prepared with Olaparib-hydroxypropyl β cyclodextrin (Kleptose) and/or Kollidon^{®} VA64 carrier.
Figure 2 shows the XRPD pattern of the pharmaceutical composition prepared with Olaparib-Kleptose and/or Kollidon^{®} VA64 carrier.
Figure 3 shows the dissolution profiles of pharmaceutical Composition 2-3 prepared with Olaparib-Kleptose and/or Kollidon^{®} VA64 mixed carrier and Olaparib API in pH 6.8 buffer.
Figure 4 shows the dissolution of Olaparib compositions in pH 6.8 buffer corresponding to different Kleptose contents in pharmaceutical compositions prepared with Olaparib-Kleptose and/or Kollidon^{®} VA64 mixed carriers (Olaparib and carrier material in a weight ratio of 1:2.3).
Figure 5 shows the mean plasma concentration-time profiles of Olaparib after administration of Composition 2-1 and Composition 2-3 prepared with Olaparib-Kleptose and/or Kollidon^{®} VA64 carrier to human subjects under fasting conditions.

### DETAILED DESCRIPTION

### Definitions

Unless defined otherwise, all the technical and scientific terms used herein have the same meanings as commonly known by a person skilled in the art. In case of confliction, the definitions provided herein will prevail. When a certain value, concentration, or the like, or a parameter is expressed in the form of a range, a preferable range, or a preferable upper limit and a preferable lower limit, it shall be appreciated that it equals to disclosure of any range obtained by combining any of the upper limits or preferable values with any of the lower limits or preferable values, regardless whether the range is specifically disclosed or not. Unless indicated otherwise, it is intended that a numerical range recited herein encompasses the end points, as well as all the integers and fractions within the range.

The terms "about" and "approximate", when used along with a numerical variable, generally means the value of the variable and all the values of the variable within an experimental error (e.g. 95% confidence interval for the mean) or within a specified value ± 10% or within a broader range.

The term "stoichiometric" means that substances are used in a certain weight ratio. For example, in the present disclosure, the active ingredient (e.g., Olaparib), the carrier material and the optional pharmaceutically acceptable pharmaceutical excipients are formulated in a specified weight ratio.

The terms "optional" or "optionally present" mean that the subsequently described event or circumstance may or may not occur, and that the description includes the occurrence and non-occurrence of said event or circumstance.

The expression "comprise" or the equivalents "include", "contain" and "have" and the like are open-ended and do not exclude additional unrecited elements, steps or ingredients. The expression "consisting of" excludes any element, step or ingredient not indicated. The expression "consisting essentially of" means that the scope is limited to the indicated elements, steps or components, plus optional elements, steps or components that do not materially affect the basic and novel characteristics of the claimed subject matter. It should be understood that the expression "comprise" encompasses the expressions "consisting essentially of" and "consisting of".

The term "one or more" may mean 1, 2, 3, 4, 5, 6, 7, 8, 9 or more.

The term "pharmaceutically acceptable" substance means that, which, according to a common medical judgment, is suitable to be in contact with a tissue of a patient without any inappropriate toxicity, irritation, allergic response, etc., has a reasonable balance between advantages and disadvantages, and can be applied to its target use effectively.

The term "pharmaceutical composition" means substances composed of one or more active ingredients, carrier and optionally one or more pharmaceutically acceptable excipients. In the present disclosure, it can be simply referred to as "composition", for example, pharmaceutical Composition 1-1 can be simply referred to as Composition 1-1.

The term "blank composition" relative to a pharmaceutical composition means it comprises no active ingredient and only comprises a carrier material and optionally one or more other pharmaceutically acceptable excipients.

The terms "pharmaceutical product", "pharmaceutical dosage form", "dosage form", "pharmaceutical formulation", etc., refer to a pharmaceutical composition administered to a patient in need of treatment, which is typically in the form of a powder, a granule, a pill, a capsule, a tablet, a solution, a suspension, or a patch, etc.

The term "dispersed at the molecular level" means that a drug is dispersed in the carrier material to form a single-phase pharmaceutical composition. In the present disclosure, the term means that Olaparib is dispersed in a carrier material to form a single-phase pharmaceutical composition (also referred to as a solid solution, dispersion or solid dispersion), the Tg value of the obtained Olaparib pharmaceutical composition is different from the Tg value of the carrier material and the API Olaparib.

The terms "dissolved in", "dispersed at a molecular level", "dispersion", "solid solution" and "solid dispersion" are used herein as appropriate to describe a pharmaceutical composition according to the disclosure in various stages during preparation and at various temperatures.

The term "bioavailability" indicates the extent to which a drug or another substance is utilized by the body after administration.

The term "peak time of plasma drug concentration (Tₘₐₓ)" means the time when peak plasma drug concentration (Cₘₐₓ) is reached after drug administration.

The term "peak plasma drug concentration (Cₘₐₓ)" means the maximum plasma drug concentration reached after drug administration.

The term "AUC_{0-∞}" means the area under a plasma drug concentration - time curve from the time point of 0 to infinity after drug administration, and the term "AUCo-t" means the area under a plasma drug concentration - time curve from the time point of 0 to t after drug administration.

"Ratio A" as used herein means the weight ratio of active ingredient to carrier material cyclodextrin (the first carrier material) in the composition or formulation, calculated on an active ingredient basis. "Ratio B" as used herein means the weight ratio of the active ingredient to the second carrier material in the composition or formulation, calculated on an active ingredient basis.

"C%" as used herein means the weight percent of the first carrier material to the sum of the first carrier material and the second carrier. For example, "C%" can be expressed as "cyclodextrin / (cyclodextrin + the second carrier)%", a specific example is Kleptose / (Kleptose + Kollidon^{®} VA64)%.

As used herein, polyvinyl caprolactam-polyvinyl acetate-polyethylene glycol graft copolymer refers to a copolymer containing vinyl caprolactam, vinyl acetate and ethylene glycol structural units, which may also be expressed as "polyethylene glycol/vinyl caprolactam/vinyl acetate copolymer".

Unless specified otherwise, all the percentages, portions and ratios, etc. in the present disclosure are on weight basis.

### Pharmaceutical composition and pharmaceutical formulation

In one aspect, provided is a pharmaceutical composition comprising an active ingredient Olaparib and a carrier material cyclodextrin. When referring to the active ingredient Olaparib, other pharmaceutically acceptable forms thereof are also encompassed, including but not limited to a pharmaceutically acceptable salt, ester, solvate (e.g., hydrate), derivative, etc. Those skilled in the art will understand that when calculating Olaparib in a composition or formulation, other forms of Olaparib (if present) should be converted into their corresponding free form.

In an embodiment, the cyclodextrin is one or more selected from the group consisting of α cyclodextrin, γ cyclodextrin, β cyclodextrin, hydroxypropyl β cyclodextrin, hydroxypropyl α cyclodextrin, sulfobutyl β cyclodextrin, and methyl cyclodextrin.

In a preferable embodiment, the cyclodextrin is hydroxypropyl β cyclodextrin. An example that can be used is, but not limited to, Kleptose, a commercially available product from Roquette, France.

In an embodiment, the weight ratio (A) of the active ingredient Olaparib to the carrier material cyclodextrin is about 1:0.5 - about 1:5, preferably about 1:0.5 - about 1:4, more preferably about 1:0.5 - about 1:3; for example, including but not limited to about 1:0.6, about 1:0.7, about 1:0.8, about 1:0.9, about 1:1, about 1:1.2, about 1:1.3, about 1:1.4, about 1:1.5, about 1:1.6, about 1:1.8, about 1:2.0, about 1:2.2, about 1:2.3, about 1:2.4, about 1:2.6, about 1:2.8, about 1:3, etc., and a range constituted by any two of these ratios (values).

In an embodiment, in addition to the carrier material cyclodextrin, the pharmaceutical composition may further comprise a second carrier material. Those skilled in the art will understand that the referenced "second" carrier material is only used to distinguish it from the carrier material cyclodextrin (also as "the first carrier material" or "the first carrier material cyclodextrin"), not in terms of nature, use or order. In a preferable embodiment, the second carrier material is one or more selected from the group consisting of copovidone, povidone, polyvinyl caprolactam-polyvinyl acetate-polyethylene glycol graft copolymer, polyoxyethylene, hypromellose and derivatives thereof, hydroxypropyl cellulose, methyl cellulose, hydroxyethyl cellulose, ethyl cellulose, sodium alginate, acrylic resin, and carbomer.

In a preferable embodiment, the second carrier material is one or more selected from the group consisting of copovidone, polyethylene glycol/vinyl caprolactam/vinyl acetate copolymer, acrylic resin, hypromellose and derivatives thereof.

In a more preferable embodiment, the second carrier material is one or more selected from the group consisting of copovidone, and polyethylene glycol/vinyl caprolactam/vinyl acetate copolymer.

In a specific embodiment, the second carrier material is polyethylene glycol/vinyl caprolactam/vinyl acetate copolymer. An example that can be used is, but not limited to, Soluplus^{®}, a commercially available product from the company BASF.

In a preferable embodiment, the second carrier material is copovidone. Examples that can be used are, but are not limited to, Kollidon^{®} VA64 commercially available from BASF or Plasdone^{™} S630 commercially available from Ashland.

In an embodiment, the weight ratio (B) of the active ingredient Olaparib to the second carrier material is about 1:0.5 - about 1:5, preferably about 1:0.5 - about 1:3, more preferably about 1:0.5 - about 1:2.5; for example, including but not limited to, about 1:0.5, about 1:0.6, about 1:0.7, about 1:0.8, about 1:0.9, about 1:1, about 1:1.1, about 1:1.2, about 1:1.3, about 1:1.5, about 1:1.6, about 1:1.7, about 1:1.9, about 1:2, about 1:2.1, about 1:2.3, about 1:2.5, etc., and a range constituted by any two of these ratios (values).

In an embodiment, the weight ratio of the first carrier material cyclodextrin to the second carrier material is about 1:1 - about 1:10, preferably about 1:1.5 - about 1:8, preferably about 1:1 - about 1:8; more preferably, about 1:1 - about 1:3, for example, about 1:1, about 1:1.5, about 1:2, about 1:3, about 1:4, about 1:5, about 1:8, about 1:10, etc., and a range constituted by any two of these ratios (values).

In an embodiment, the weight ratio of the active ingredient Olaparib, the first carrier material cyclodextrin, and the second carrier material is about 1:0.5:0.5 - about 1:2.0:3.0, preferably about 1:0.5:0.5 - about 1:1.5:2.5, more preferably about 1:0.5:0.5 - about 1:1:2.2; for example, including but not limited to, about 1:0.6:1.7, about 1:0.7:1.6, about 1:0.8:1.5, about 1:0.9:0.9, about 1:1:1, about 1:1.1:1.9, about 1:1.2:1.8, about 1:1.5:1.5, about 1:1.0:2, etc., and a range constituted by any two of these ratios (values).

In an embodiment, the ratio of the active ingredient Olaparib to the total weight of carrier material (i.e., the first carrier or the first carrier plus the second carrier) is about 1:0.5 - about 1:5, preferably about 1:0.5 - about 1:4, more preferably about 1:0.5 - about 1:3, for example, including but not limited to about 1:0.6, about 1:0.7, about 1:0.8, about 1:0.9, about 1:1, about 1:1.1, about 1:1.2, about 1:1.3, about 1:1.4, about 1:1.5, about 1:1.6, about 1:1.7, about 1:1.8, about 1:1.9, about 1:2.0, about 1:2.1, about 1:2.2, about 1:2.3, about 1:2.4, about 1:2.5, about 1:2.6, about 1:2.7, about 1:2.8, about 1:2.9, about 1:3, etc., and a range constituted by any two of these ratios (values).

In embodiments of the present disclosure, C% may be about 9%-50%, preferably about 11%-50%, more preferably about 29%-50%, for example, about 17%, about 20%, about 25%, about 33%, about 50%, etc., and a range constituted by any two of these ratios (values).

In an embodiment, Olaparib is dissolved in the carrier material or dispersed at the molecular level in the carrier material. It should be understood that the carrier material refers to the first carrier material or to the first and second carrier materials (if present).

In another embodiment, the pharmaceutical composition may further comprise a solubilizer. The solubilizer is preferably polyethylene glycol 1000 vitamin E succinate (D-α-tocopherol polyethylene glycol 1000 succinate, also known as TPGS, Vitamin E TPGS or Tocophersolan). The TPGS applicable in the present disclosure is a water-soluble derivative of vitamin E, which is formed by esterification of the carboxyl group of vitamin E succinate (VES) and polyethylene glycol (PEG) 1000, with a relative molecular weight of about 1513, which has been included in the United States Pharmacopoeia. TPGS not only acts as a solubilizer in the pharmaceutical composition and formulation of the present disclosure, but can also contribute to increased bioavailability by affecting the efflux of the drug. An example of a useful TPGS is but not limited to, Kolliphor^{™} TPGS, a commercially available product from BASF.

In the pharmaceutical composition of the present disclosure, the amount of the solubilizer used is not particularly limited and can be adjusted according to the actual situation. Generally, the weight ratio of the solubilizer to the total weight of the active ingredient Olaparib, the first carrier material cyclodextrin and/or the second carrier material and the TPGS is about 0.5-12%, preferably about 7-11%.

In yet another aspect, provided is a pharmaceutical formulation comprising the pharmaceutical composition of the present disclosure.

In an embodiment, the pharmaceutical formulation and/or pharmaceutical composition of the present disclosure also comprise a pharmaceutically acceptable excipient. Those skilled in the art will understand that the pharmaceutically acceptable excipient is used to assist the preparation and improve the dosage form (for example, stability, uniformity, etc.), and will not substantially change the properties of the pharmaceutical composition/formulation of the present disclosure, especially the active ingredient thereof. Those skilled in the art can adjust the type and/or content of the excipient according to actual needs, without affecting the properties of the pharmaceutical composition/formulation of the present disclosure.

In an embodiment, the pharmaceutically acceptable excipient includes but not limited to one or more of a glidant, a surfactant, a pH adjusting agent, a diluent, a disintegrant, a binder, and a lubricant.

As an example, the glidant may be one or more of colloidal silicon dioxide and talcum.

As an example, the surfactant may be one or more of polyoxyethylene castor oil derivatives, polyoxyethylene glyceryl stearate, polyethylene glycol castor oil derivatives, block copolymer of ethylene oxide and propylene oxide, mono-fatty acid ester of polyoxyethylene (20) sorbitan, polyethylene glycol fatty acid ester, alkylene glycol fatty acid monoester and sorbitan fatty acid monoester.

As an example, the pH adjusting agent may be one or more of citric acid, acetic acid, fumaric acid, maleic acid, tartaric acid, malic acid, succinic acid, oxalic acid, malonic acid, benzoic acid, mandelic acid and ascorbic acid, preferably citric acid.

As an example, the diluent may be one or more of microcrystalline cellulose, starch, pregelatinized starch, lactose, mannitol, calcium hydrogen phosphate.

As an example, the disintegrant may be one or more of microcrystalline cellulose, carboxymethyl cellulose, crospovidone, croscarmellose sodium, carboxymethyl cellulose calcium, sodium carboxymethyl starch, low-substituted hydroxypropyl cellulose, hydroxymethyl starch, alginic acid, sodium alginate, guar gum, corn starch and magnesium aluminum silicate, preferably croscarmellose sodium.

As an example, the binder may be one or more of polyethylene glycol, starch, hydroxypropyl cellulose, hydroxypropyl methylcellulose, pregelatinized starch, polyvinylpyrrolidone, acacia powder and gelatin, preferably starch, pregelatinized starch, modified starch, microcrystalline cellulose, silicified microcrystalline cellulose, low-substituted hydroxypropyl cellulose, glucose, sucrose, lactose, sorbitol, mannitol, erythritol, calcium carbonate and calcium hydrogen phosphate, preferably one or more of mannitol, microcrystalline cellulose and silicified microcrystalline cellulose.

As an example, the lubricant may be one or more of magnesium stearate, stearic acid, stearate, sodium stearyl fumarate, sodium lauryl sulfate, polyethylene glycol, sodium benzoate, sucrose fatty acid esters, micronized silica gel, talcum, glyceryl monostearate, glyceryl behenate, glyceryl palmitostearate, stearic acid and hydrogenated vegetable oil.

Those skilled in the art will understand that the pharmaceutically acceptable excipients listed above are only illustrative and representative. Therefore, the pharmaceutical formulation of the present disclosure is not limited to include only the pharmaceutically acceptable excipients listed above. Those skilled in the art can make various changes, adjustments or equivalent replacements to the above-mentioned excipients according to conventional techniques without extending beyond the scope of the present disclosure. Those skilled in the art will also understand that the pharmaceutical composition of the present disclosure may also contain pharmaceutically acceptable excipients, such as those described above.

The pharmaceutical composition of the present disclosure can be further combined with a pharmaceutically acceptable pharmaceutical excipient to prepare various dosage forms as required. In an embodiment, the pharmaceutical formulation may be in the form of a powder, a granule, a pill, a capsule or a tablet.

### Preparation process of the present disclosure

In an embodiment of the present disclosure, provided is a process for preparing the pharmaceutical composition of the present disclosure, comprising:
1) providing the active ingredient Olaparib and carrier material;
2) adding the materials of step 1) into a hot melt extruder and performing extruding at about 120°C-190°C;
3) cooling, pulverizing and sieving the extrudate obtained in step 2) to obtain the pharmaceutical composition.

Optionally, the carrier material referred to in step 1) may include cyclodextrin and may further include a second carrier material. Optionally, a pharmaceutically acceptable pharmaceutical excipient can be added in step 1) and/or step 2). The addition of these materials can be carried out according to the desired stoichiometric ratio.

The temperature in step 2) may be 120°C-190°C, e.g., about 170-190°C, about 160-190°C, or 140-about 180°C. Optionally, the hot melt extruder can be preheated to the above-mentioned temperature before or at the same time as the above-mentioned steps are carried out.

The hot melt extruder could be fed with a blend of Olaparib, carrier material and optional pharmaceutically acceptable excipient that had been mixed (or fed with a blend of Olaparib, carrier material, TPGS, and optional pharmaceutically acceptable excipient in stoichiometric ratio that had been mixed), or a stoichiometric ratio of Olaparib, carrier material, and optional pharmaceutically acceptable pharmaceutical excipient (or directly fed with Olaparib, carrier material, TPGS and optional pharmaceutically acceptable pharmaceutical excipient) could also be directly fed into the hot melt extruder. Extrusion is then performed, and the resulting extrudate is cooled, pulverized and sieved, optionally mixed with a pharmaceutically acceptable pharmaceutical excipient, thereby the pharmaceutical composition is obtained.

The cooling used is not particularly limited, and may include air cooling, water cooling, mechanical cooling, and the like.

The type of extruder suitable for use in the present disclosure is not particularly limited and includes, but not limited to, single-screw or twin-screw type hot melt extruders. In an embodiment, the extruder used to prepare the pharmaceutical composition of the present disclosure is a twin-screw type extruder. In this case, the type of screw rotation is not particularly limited and includes, but is not limited to, co-rotating twin-screw, counter-rotating twin-screw, and double-cone screw rotation mode.

In a preferable embodiment, the extruder used to prepare the pharmaceutical composition of the present disclosure is preferably a co-rotating twin-screw type extruder. The temperature set for the hot melt extruder is about 160-190°C, and the screw speed is about 120-180 rpm. The ratio of screw length to diameter (L/D) can be selected from about 25-40. If the temperature of the hot melt extruder is too low, the L/D is too short, and the screw speed is too slow, the thermal energy and mechanical energy will be insufficient during the hot melt process so that Olaparib, the carrier material or TPGS cannot reach a molten state, or Olaparib cannot be dissolved in the molten carrier material. Therefore, although Olaparib is mixed well with the carrier material, a single-phase solid dispersion (solid solution) in which Olaparib is dissolved or dispersed at the molecular level in the carrier material cannot be obtained. If the temperature of the hot melt extruder is too high, the L/D is too long, and the screw speed is too fast, the thermal energy and mechanical energy will be provided excessively during the hot melt process. Even if a single-phase solid dispersion (solid solution) of Olaparib dissolved or dispersed at the molecular level in the carrier material is obtained, undesirable degradation of Olaparib and/or carrier material and/or TPGS may also occur.

In other embodiments, the pharmaceutical composition of the present disclosure, in addition to the hot melt extrusion, can also be prepared by, for example, a solution evaporation process, a spray drying process, or a coprecipitation process. These processes are also within the scope of the present disclosure.

### Beneficial effect

In one aspect, provided is a pharmaceutical composition comprising Olaparib, which increases the absorption and bioavailability of the active ingredient Olaparib *in vivo,* reduces energy consumption for production, and improves production efficiency. In particular, in the pharmaceutical composition of the present disclosure, the active ingredient Olaparib is dispersed in the cyclodextrin carrier material or a monolithic carrier material (in the case of inclusion of a second or other carrier material), which can improve the solubility of Olaparib in the gastrointestinal tract, increase the absorption of Olaparib in the body, and improve the bioavailability *in vivo.*

On the other hand, the pharmaceutical composition of the present disclosure also has better production process characteristics, such as easy grinding, better compressibility, short disintegration time, etc., so that production energy consumption is reduced, and production efficiency is increased.

Further, using the combination of the first carrier material cyclodextrin and the second carrier material (e.g., polyethylene glycol/vinyl caprolactam/vinyl acetate copolymers such as Soluplus, and/or copovidone, such as Kollidon VA64 or Ashland Plasdone S630) as the mixed carrier material in the pharmaceutical composition of the present disclosure can further improve the solubility of Olaparib in the gastrointestinal tract, thereby further improving the absorption and bioavailability of Olaparib.

Furthermore, the addition of polyethylene glycol 1000 vitamin E succinate (TPGS) to the pharmaceutical composition can further improve the solubility of Olaparib in the gastrointestinal tract, thereby further increasing the absorption and bioavailability of Olaparib. In addition, when the pharmaceutical composition is prepared by the hot melt extrusion process of the present disclosure, the addition of TPGS can significantly reduce the hot melt extrusion operating temperature of the pharmaceutical composition, significantly reduce torque, reduce energy consumption, and improve production capacity.

### Examples

Unless specified otherwise, the reagents and raw materials used herein are commercially available. For example, API (active ingredient) Olaparib can be purchased from ScinoPharm, Ltd. Taiwan; hydroxypropyl β-cyclodextrin can be purchased from Roquette Company, France; polyethylene glycol/vinyl caprolactam/vinyl acetate copolymer and TPGS can be purchased from BASF; and copovidone can be purchased from BASF or Ashland.

Examples of assays used in the evaluation of physicochemical properties in each embodiment are as follows:
1. Glass transition temperature (Tg): more than 3 mg of the analyte (e.g., API Olaparib (hereinafter referred to as API) and drug-loaded composition (i.e., the pharmaceutical composition of the present disclosure) or blank composition) were precisely weighed for modulated differential scanning calorimetry (mDSC, TA Q2000 differential scanning calorimeter), and the scanning temperature range was 40-180°C.
2. Powder X-ray Diffraction (XRPD): an appropriate amount of the substance (API, drug-loaded composition or blank composition) was taken for testing, and the powder X-ray diffraction pattern (D8ADVANCE X-ray diffractometer by BRUKER) was recorded under the conditions of Cu target, voltage 45 kv, and current 45 mA.
3. Apparent solubility: excess amount of Olaparib pharmaceutical composition was weighed into a container, about 2/3 of the volume of the container with pH 6.8 phosphate buffer was added, and the container was placed in a shaker at 37°C for 2 h. After filtering the contents of the container with a 0.45 µm filter membrane, the filtrate was collected, diluted with an appropriate amount of acetonitrile, mixed by vortex, and the concentration of Olaparib was determined by HPLC analysis. The HPLC analysis procedure was as follows:

| Column | Waters Xselect C18 3.5 µm.150×4.6 mm |
|---|---|
| Mobile phase | Mobile phase A: 0.05% TFA water/ |
| | Mobile phase B: 0.05% TFA acetonitrile |
| Flow rate | 1.0 mL/min |
| Sample tray | Room temperature |
| Detection wavelength | 276 nm |
| Injection volume | 10 µL |
| Single needle analysis time | about 10 min |

4. Dissolution

| | |
|---|---|
| Dissolution method | USP Method I (Basket Method) |
| Dissolution medium | Phosphate buffer pH 6.8: 900 mL |
| Rotating speed | 100 rpm |
| Temperature | 37.5 °C |
| Testing dose | 150 mg (Olaparib)/cup |

5. Dissolution sample analysis: the same as the HPLC analysis method described in the above-mentioned apparent solubility assay.

### Example 1 Olaparib-cyclodextrin pharmaceutical composition

### 1. Preparation

Compositions of the Olaparib-cyclodextrin pharmaceutical composition and the amount of each component were shown in Table 1-1.

Preparation: Olaparib, carrier material cyclodextrin (Hydroxypropyl β cyclodextrin, Kleptose) and/or TPGS and other excipient ingredients according to the amount shown in

Table 1-1 were fed into the hopper of a co-rotating twin-screw extruder (Omicron 12, Steer Corporation, India) directly or after uniform mixing in a mixer. The temperature of the co-rotating twin-screw extruder was kept in the range of about 170°C - about 190°C, and the extrusion was carried out at a screw speed of about 120 rpm - about 170 rpm. The resulting extrudate was cooled, pulverized and sieved to obtain the pharmaceutical composition.

**Table 1-1 Composition of the Olaparib-cyclodextrin pharmaceutical composition and the amount of each component (% by weight)**

| Ingredients | Function | Composition 1-1 | Composition 1-2 | Composition 1-3 | Composition 1-4 | Composition 1-5 | Composition 1-6 |
|---|---|---|---|---|---|---|---|
| Olaparib | Active ingredient | 18.8 | 18.0 | 16.4 | 17.5 | 11.3 | 9.4 |
| Kleptose | Carrier material | 33.8 | 41.5 | 37.7 | 52.5 | 45.1 | 47.0 |
| Kolliphor^{®} TPGS | Solubilizer | / | / | 5.4 | / | / | / |
| Colloidal silicon dioxide | Glidant | 1.4 | 1.4 | 1.4 | 1.4 | 1.4 | 1.6 |
| Mannitol | Diluent | 39.0 | 30.0 | 30.0 | 19.5 | 35.2 | 32.9 |
| Croscarmellose sodium | Disintegrant | 6.0 | 8.3 | 8.3 | 8.3 | 6.0 | 8.3 |
| Magnesium stearate | Lubricant | 1.0 | 0.8 | 0.8 | 0.8 | 1.0 | 0.8 |

### 2. Evaluation of physicochemical properties

### 2.1. The glass transition temperature (Tg) of the pharmaceutical composition was determined.

The test results are shown in Table 1-2.

**Table 1-2 Glass transition temperature (Tg) of Olaparib-cyclodextrin pharmaceutical composition**

| Formula | API | Composition 1-1 | Composition 1-2 | Blank Composition 1-2 | Composition 1-3 | Blank Composition 1-3 | Composition 1-4 | Composition 1-5 | Composition 1-6 |
|---|---|---|---|---|---|---|---|---|---|
| mDSC (Onset, Tg °C) | 90.2 | 105.2 | 110.3 | 99.2 | 102.8 | 93.6 | 114.5 | 116.6 | 119.7 |
| Melting point (°C) | 210.4 | / | | | | | | | |

As can be seen from Table 1-2, compared with the corresponding blank composition, the Tg values of the pharmaceutical compositions were obviously shifted, and the melting peak of Olaparib had disappeared, which was different from the Tg value of Olaparib (90.2 °C). The above results clearly showed that in each pharmaceutical composition of the present disclosure, Olaparib was dissolved in the carrier material or dispersed in the carrier material at the molecular level, forming a molecularly dispersed solid dispersion (i.e., a glass solution).

### 2.2. Apparent solubility of pharmaceutical compositions

The test results are shown in Table 1-3.

**Table 1-3 Apparent solubility of Olaparib-cyclodextrin pharmaceutical composition in pH 6.8 buffer**

| Items | API | Composition 1-1 | Composition 1-2 | Composition 1-3 | Composition 1-4 | Composition 1-5 | Composition 1-6 |
|---|---|---|---|---|---|---|---|
| Apparent Solubility (mg/ml) | <0.1 | 0.42 | 0.47 | 0.68 | 0.53 | 0.61 | 0.60 |
| Solubility ratio (Composition/API) | 1 | >3 | >4 | >6 | >5 | >6 | >6 |

It can be seen from Table 1-3 that Olaparib showed obvious solubilizing effect in each pharmaceutical composition of the present disclosure, indicating that cyclodextrin had good solubilizing effect on Olaparib.

Adjusting the weight ratio of API (i.e., active ingredient) and carrier material cyclodextrin (Kleptose) in the pharmaceutical composition from 1:1.8 (Composition 1-1) to 1:5 (Composition 1-6) could increase the apparent solubility from 0.42 mg/ml to 0.60 mg/ml. It can be seen that the change in apparent solubility was limited, indicating that the weight ratio of active ingredient to carrier material cyclodextrin had little effect on the solubility of Olaparib, and when the carrier material was increased to a certain extent (Composition 1-5 and Composition 1-6), the solubility remained basically unchanged.

However, addition of a small amount of TPGS (composition 1-3) to composition 1-2 increased the apparent solubility from 0.47 mg/ml to 0.68 mg/ml, indicating that the pharmaceutical composition containing TPGS could greatly improve the solubility of Olaparib under the condition of reduced the amount of carrier material.

### 2.3 Dissolution of pharmaceutical compositions

The main absorption site of drugs in the human body is the small intestine, and the pH value in the small intestine is about 6.8. According to the method described above, the dissolution of each Olaparib-cyclodextrin pharmaceutical composition in pH 6.8 phosphate buffer was determined, and the results were shown in Table 1-4.

**Table 1-4 Dissolution of Olaparib-cyclodextrin pharmaceutical composition in pH 6.8 phosphate buffer**

| Formula | Cumulative dissolution in pH 6.8 phosphate buffer (%) | | | | | |
|---|---|---|---|---|---|---|
| | 15 min | 30 min | 45 min | 60 min | 90 min | 120 min |
| API | 12.2 | 21.4 | 30.2 | 37.5 | 39.8 | 40.2 |
| Composition 1-1 | 20.7 | 32.6 | 50.8 | 60.9 | 74.1 | 85.8 |
| Composition 1-2 | 26.1 | 41.7 | 58.4 | 69.6 | 82.3 | 94.5 |
| Composition 1-3 | 36.8 | 52.7 | 77.5 | 86.2 | 95.4 | 101.3 |
| Composition 1-4 | 31.1 | 45.2 | 64.1 | 73.6 | 85.3 | 96.5 |
| Composition 1-5 | 34.1 | 48.9 | 73.1 | 82.6 | 90.3 | 97.2 |
| Composition 1-6 | 32.3 | 46.7 | 70.5 | 78.1 | 88.9 | 96.8 |

According to the data in the above table, it can be seen that the dissolution of the API within 2 h was only 40.2%. Compared with API, the dissolution of the pharmaceutical composition of the present disclosure in the pH 6.8 phosphate buffer solution at the end point of 2 h was higher than 85%, and the dissolution of all the pharmaceutical compositions of the present disclosure at each time point after 30 min was all above 50%.

In particular, the dissolution of Olaparib-cyclodextrin pharmaceutical Composition 1-3 containing TPGS reached the highest 101.3% at the end point of 2 h, which was significantly higher than that of the API, indicating that the pharmaceutical compositions of the present disclosure could significantly improve the absorption of Olaparib *in vivo.* In addition, the dissolution of pharmaceutical Composition 1-3 at each time point after 30 min was all above 75%. This indicated that in the pharmaceutical compositions of the present disclosure, the absorption of Olaparib *in vivo* could be better improved by the addition of solubilizer.

### Example 2 Olaparib-(mixed) carrier pharmaceutical composition

### 1. Preparation

Compositions of the Olaparib-(mixed) carrier pharmaceutical composition and the amount of each component were shown in Table 2-1.

Preparation: Olaparib and carrier material (cyclodextrin (Kleptose) and/or second carrier (Kollidon^{®} VA64)) and/or TPGS and other excipient ingredients according to the amount shown in Table 2-1 were fed into the hopper of a co-rotating twin-screw extruder (Omicron 12, Steer Corporation, India) directly or after uniform mixing in a mixer. The temperature of the co-rotating twin-screw extruder was kept in the range of about 160 - about 190°C, and the extrusion was carried out at a screw speed of about 120 - about 180 rpm. The resulting extrudate was cooled, pulverized and sieved to obtain the pharmaceutical composition.

**Table 2-1 Compositions of the Olaparib-(mixed) carrier pharmaceutical composition and the amount of each component (% by weight)**

| Ingredients | Function | Composition 2-1 | Composition 2-2 | Composition 2-3 | Composition 2-4 | Composition 2-5 | Composition 2-6 |
|---|---|---|---|---|---|---|---|
| Olaparib | Active ingredient | 22.5 | 17.9 | 16.5 | 16.5 | 17.3 | 15.5 |
| Kollidon^{®} VA64 | Carrier material | 51.8 | 30.9 | 25.4 | 19.0 | 20.8 | 30.0 |
| Kleptose | Carrier material | / | 10.3 | 12.7 | 19.0 | 10.4 | 15.0 |
| Kolliphor^{®} TPGS | Solubilizer | 7.4 | 5.9 | 5.5 | 5.5 | 4.8 | 6.0 |
| Colloidal silicon dioxide | Glidant | 1.8 | 1.4 | 1.3 | 1.3 | 1.2 | 1.5 |
| Mannitol | Diluent | 15.5 | 26.4 | 32.2 | 32.3 | 37.3 | 24.2 |
| Croscarmellose sodium | Disintegrant | / | 6.2 | 5.4 | 5.4 | 7.2 | 6.8 |
| Sodium octadecyl fumarate | Lubricant | 1.0 | 1.0 | 1.0 | 1.0 | 1.0 | 1.0 |

### 2. Evaluation of physicochemical properties

### 2.1. Glass transition temperature (Tg)

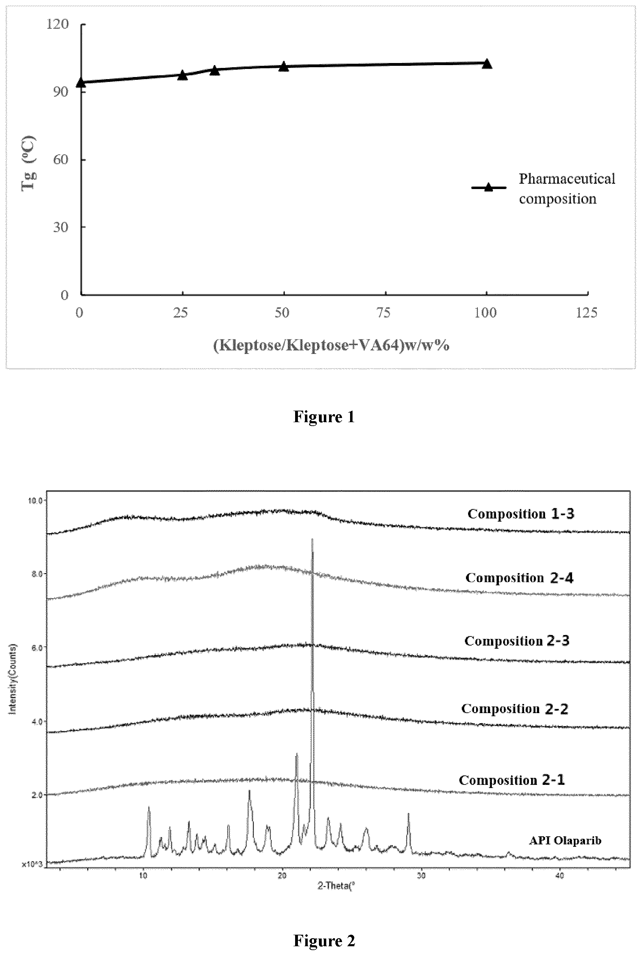

The glass transition temperature testing results of the Olaparib-(mixed) carrier pharmaceutical composition were shown in Table 2-2 and Figure 1. Figure 1 showed the effect of the content of cyclodextrin (Kleptose) in the pharmaceutical composition on the Tg value. While maintaining the same weight ratio of API (i.e., active ingredient) and (total) carrier material (e.g. API: carrier material = 1:2.3), when the proportion of cyclodextrin in the total carrier material C% (cyclodextrin/(cyclodextrin + second carrier)%, which could be calculated as Kleptose/(Kleptose + Kollidon^{®} VA64)%) is 0%, 25%, 33%, 50% and 100%, which correspond to Composition 2-1, Composition 2-2, Composition 2-3, Composition 2-4, and Composition 1-3, respectively. As can be seen from Figure 1, with the increase of cyclodextrin Kleptose content in the composition, the Tg value showed an upward trend, and it was significantly different from the Tg value of Olaparib (90.2 °C), and the melting peak of Olaparib had disappeared. Thus, in each Olaparib-(mixed) carrier pharmaceutical composition of the present disclosure, Olaparib was dissolved in the carrier material or dispersed in the carrier material at the molecular level, forming a molecularly dispersed solid dispersion (i.e., a glass solution).

**Table 2-2 Glass transition temperature (Tg) of Olaparib-(mixed) carrier pharmaceutical composition**

| Formula | API | Composition 2-1 | Composition 2-2 | Composition 2-3 | Composition 2-4 | Composition 2-5 | Composition 2-6 |
|---|---|---|---|---|---|---|---|
| mDSC (Onset, Tg °C) | 90.2 | 94.2 | 97.6 | 99.8 | 101.3 | 96.1 | 105.2 |
| Melting point (°C) | 210.4 | / | | | | | |

### 2.2. Powder-X-ray diffraction characterization (XRPD pattern)

Figure 2 showed the XRPD pattern of the pharmaceutical composition prepared with Olaparib-cyclodextrin (Kleptose) and/or a second carrier (Kollidon^{®} VA64), and the XRPD patterns, from bottom to top, were the API, Composition 2-1, Composition 2-2, Composition 2-3, Composition 2-4 and Composition 1-3. It can be seen from Figure 2 that the crystal diffraction peaks of Olaparib were not seen in the XRPD patterns of Composition 1-3 and Composition 2-1 (from single carrier material), and Composition 2-2, Composition 2-3 and Composition 2-4 (from mixed carrier materials), indicating that in the pharmaceutical compositions of the present disclosure, Olaparib was dissolved in the carrier material or dispersed in the carrier material at the molecular level.

### 2.3. Apparent solubility

**Table 2-3 Apparent solubility of Olaparib-(mixed) carrier pharmaceutical composition in pH 6.8 buffer**

| Items | API | Composition 2-1 | Composition 2-2 | Composition 2-3 | Composition 2-4 | Composition 2-5 | Composition 2-6 |
|---|---|---|---|---|---|---|---|
| Apparent Solubility (mg/mL) | <0.1 | 0.63 | 0.94 | 0.96 | 0.82 | 0.73 | 1.01 |
| Solubility ratio (Composition/API) | 1 | >6 | >9 | >9 | >8 | >7 | >10 |

It can be seen from Table 2-3 that each pharmaceutical composition of the present disclosure showed obvious solubilizing effect on Olaparib, wherein the solubility of Olaparib was improved most obviously by Composition 2-6.

In one aspect, the pharmaceutical composition prepared by mixing carrier materials could improve the solubility of Olaparib within a certain range. For example, when the weight ratio of API (i.e., active ingredient) and (total) carrier material was kept constant (e.g. 1:2.3), the C% was adjusted from 100% (Composition 1-3) to 50% (Composition 2-4), the apparent solubility increased from 0.68 mg/ml to 0.82 mg/ml; when C% was further adjusted from 50% (Composition 2-4) to 33% (Composition 2-3), the apparent solubility continued to increase from 0.82 mg/ml to 0.96 mg/ml; when C% was further adjusted from 33% (Composition 2-3) to 25% (Composition 2-2), the apparent solubility decreased from 0.96 mg/ml to 0.94 mg/ml, with no significant change. However, when C% was further reduced to 0% (Composition 2-1), the apparent solubility was reduced to 0.63 mg/ml. This indicated that the pharmaceutical composition prepared by mixing carrier materials could improve the solubility of Olaparib within a certain range.

On the other hand, mixed carrier materials could significantly improve the apparent solubility of Olaparib. For example, when C% was kept constant (e.g. 33%) in the mixed carrier material, when the weight ratio of API (i.e. active ingredient) and (total) carrier material was adjusted from 1:1.8 (Composition 2-5) to 1:2.3 (Composition 2-3) and 1:3 (Composition 2-6), the apparent solubility of the pharmaceutical compositions were at least 7, 9 and 10 times higher than that of the API, respectively, and were higher than the composition of single carrier material, that is, the apparent solubility of the composition containing only single carrier cyclodextrin (Kleptose) (Composition 1-3) and only single second carrier (VA64) (Composition 2-1). This indicated that the use of mixed carrier materials could significantly improve the apparent solubility of Olaparib, and further improve the absorption thereof *in vivo,* namely, the bioavailability, compared to the use of single carrier material. For example, when C% in the mixed carrier material was 33%, the corresponding compositions (i.e., Composition 2-3, Composition 2-5 and Composition 2-6) all had obvious solubilizing effect on Olaparib.

### 2.4. Dissolution

**Table 2-4 Dissolution of Olaparib-(mixed) carrier pharmaceutical composition in pH 6.8 phosphate buffer**

| Formula | Cumulative dissolution in pH 6.8 phosphate buffer (%) | | | | | |
|---|---|---|---|---|---|---|
| | 15 min | 30 min | 45 min | 60 min | 90 min | 120 min |
| API | 12.2 | 21.4 | 30.2 | 37.5 | 39.8 | 40.2 |
| Composition 2-1 | 35.6 | 50.2 | 75.6 | 84.2 | 92.1 | 98.4 |
| Composition 2-2 | 47.5 | 78.8 | 93.1 | 98.6 | 102.3 | 101.5 |
| Composition 2-3 | 48.8 | 80.2 | 96.5 | 99.6 | 101.3 | 102.1 |
| Composition 2-4 | 43.2 | 74.8 | 90.4 | 94.9 | 98.4 | 101.2 |
| Composition 2-5 | 39.2 | 62.8 | 85.2 | 90.4 | 98.2 | 100.6 |
| Composition 2-6 | 51.3 | 85.2 | 99.6 | 101.1 | 102.2 | 101.8 |

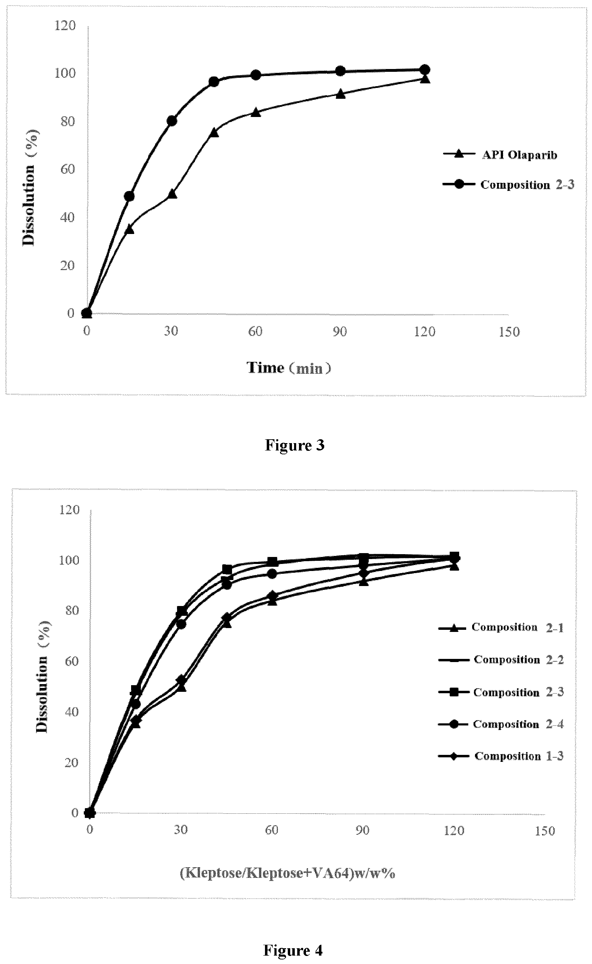

The dissolution profiles of Composition 2-3 and API were shown in Figure 3. It can be seen from Table 2-4 and Figure 3 that the dissolution of the API within 2 h at pH 6.8 was about 40%.

Compared with API:
1) The dissolution of the pharmaceutical compositions of the present disclosure in pH 6.8 within 2h was significantly improved, wherein the dissolution of Composition 2-2 to Composition 2-6 at each time point after 30 min was all above 85%, indicating that it could significantly improve the absorption of Olaparib *in vivo.*
2) The dissolution of Composition 2-2 to Composition 2-6 prepared with mixed carrier material was higher than that of Composition 2-1 and Composition 1-3 prepared with single carrier material, therefore, the overall absorption of the Composition 2-2 to the Composition 2-6 prepared with mixed carrier material in the body was better than those of the Composition 2-1 and the Composition 1-3.

Figure 4 showed the effect of cyclodextrin content on the dissolution of Olaparib at pH 6.8 in the pharmaceutical composition prepared with Olaparib-(mixed) carrier (weight ratio of Olaparib and carrier material was 1:2.3), wherein the dissolution when C% was 0%, 25%, 33%, 50% and 100% correspond to those of Composition 2-1, Composition 2-2, Composition 2-3, Composition 2-4 and Composition 1-3, respectively. It can be seen from Figure 4, at pH 6.8, the dissolution of the compositions prepared with mixed carrier materials was improved compared with Compositions 2-1 and 1-3 prepared with single carrier material, wherein when C% was 25% and 33%, respectively, the dissolution of Composition 2-2 and Composition 2-3 prepared with the corresponding mixed carrier material improved most significantly, and the dissolution of both compositions at pH 6.8 was significantly better than Composition 2-1 containing only the second carrier (Kollidon^{®} VA64) and Composition 1-3 containing only cyclodextrin (Kleptose).

### Example 3 Olaparib-(mixed) carrier pharmaceutical composition

### 1. Preparation

Compositions of the Olaparib-(mixed) carrier pharmaceutical composition and the amount of each component were shown in Table 3-1.

**Table 3-1 Compositions of the Olaparib-(mixed) carrier pharmaceutical composition and the amount of each component (% by weight)**

| Ingredients | Function | Composition 3-1 | Composition 3-2 | Composition 3-3 | Composition 3-4 | Composition 3-5 | Composition 3-6 |
|---|---|---|---|---|---|---|---|
| Olaparib | Active ingredient | 8.8 | 8.0 | 16.5 | 15.0 | 16.5 | 16.5 |
| Soluplus^{®} | Carrier material | 26.3 | 23.9 | 37.1 | 33.7 | 33.0 | 24.7 |
| Kleptose | Carrier material | / | / | 12.4 | 11.3 | 16.5 | 24.8 |
| Kolliphor^{®} TPGS | Solubilizer | / | 3.2 | / | 6.0 | / | / |
| Colloidal silicon dioxide | Glidant | 1.4 | 1.4 | 2.6 | 2.6 | 2.6 | 2.6 |
| microcrystalline cellulose | Diluent | 56.7 | 56.7 | 24.9 | 24.9 | 24.9 | 24.9 |
| Croscarmellose sodium | Disintegrant | 5.8 | 5.8 | 5.5 | 5.5 | 5.5 | 5.5 |
| Magnesium stearate | Lubricant | 1.0 | 1.0 | 1.0 | 1.0 | 1.0 | 1.0 |

Preparation: Olaparib, mixed carrier materials (Soluplus^{®} and Kleptose) and other excipient ingredients according to the amount shown in Table 3-1 were fed into the hopper of a co-rotating twin-screw extruder (Omicron 12, Steer Corporation, India) directly or after uniform mixing in a mixer. The temperature of the co-rotating twin-screw extruder was kept in the range of about 140 - about 180°C, and the extrusion was carried out at a screw speed of about 110 - about 180 rpm. The resulting extrudate was cooled, pulverized and sieved to obtain the solid powder. Then the other pharmaceutical excipients and the solid powder were mixed uniformly according to the dose shown in Table 3-1 to obtain the Olaparib pharmaceutical composition.

### 2. Evaluation of physicochemical properties

### 2.1. Apparent solubility of pharmaceutical compositions

**Table 3-2 Apparent solubility of Olaparib-(mixed) carrier pharmaceutical composition in pH 6.8 buffer**

| Items | API | Composition 3-1 | Composition 3-2 | Composition 3-3 | Composition 3-4 | Composition 3-5 | Composition 3-6 |
|---|---|---|---|---|---|---|---|
| Apparent Solubility (mg/ml) | <0.1 | 0.52 | 0.74 | 0.72 | 0.87 | 0.63 | 0.58 |
| Solubility ratio (Composition/API) | 1 | >5 | >7 | >7 | >8 | >6 | >5 |

It can be seen from Table 3-2 that the Olaparib mixed carrier pharmaceutical composition could significantly improve the apparent solubility of Olaparib and was better than the composition containing only single carrier material. For example, when the weight ratio of API and mixed carrier material was kept constant (e.g., 1:3), each Olaparib-(mixed) carrier pharmaceutical composition (3-3, 3-5, 3-6) could significantly improve the apparent solubility of Olaparib and was better than the Composition 3-1 containing only single carrier material, and the improvement of the Composition 3-3 was the most significant. This indicated that use of mixed carrier materials could significantly improve the apparent solubility of Olaparib, and further improve the absorption thereof *in vivo,* compared to the use of single carrier material.

On the other hand, the addition of solubilizer could improve solubility. For example, when the weight ratio of API and carrier material was kept constant (e.g., 1:3), after a small amount of TPGS on the basis of Composition 3-1 and Composition 3-3 was added respectively (corresponding to Composition 3-2 and Composition 3-4), the apparent solubility increased from 0.52 mg/ml and 0.72 mg/ml to 0.74 mg/ml and 0.87 mg/ml, respectively. This indicated that the pharmaceutical composition containing the solubilizer could further improve the absorption of Olaparib *in vivo.*

### 2.2 Dissolution of pharmaceutical compositions

**Table 3-3 Dissolution of Olaparib-Soluplus^{®}/ Kleptose pharmaceutical composition in pH 6.8 phosphate buffer**

| Formula | Cumulative dissolution in pH 6.8 phosphate buffer (%) | | | | | |
|---|---|---|---|---|---|---|
| | 15 min | 30 min | 45 min | 60 min | 90 min | 120 min |
| API | 12.2 | 21.4 | 30.2 | 37.5 | 39.8 | 40.2 |
| Composition 3-1 | 29.4 | 44.7 | 62.4 | 71.4 | 83.3 | 94.5 |
| Composition 3-2 | 38.4 | 61.4 | 81.5 | 88.9 | 96.2 | 99.6 |
| Composition 3-3 | 36.4 | 58.4 | 78.5 | 86.9 | 95.8 | 99.2 |
| Composition 3-4 | 44.5 | 76.2 | 91.2 | 96.3 | 100.1 | 101.3 |
| Composition 3-5 | 34.6 | 48.8 | 73.2 | 82.2 | 90.8 | 99 |
| Composition 3-6 | 32.2 | 46.7 | 68.6 | 76.7 | 87.3 | 98.7 |

It can be seen from Table 3-3, compared with the API:
1) The dissolution of the pharmaceutical composition of the present disclosure in pH 6.8 within 2 h was significantly improved, where the improvement of Composition 3-4 was the most significant, the dissolution at each time point after 30 min was all above 90%, and the dissolution of the Composition 3-3 and 3-4 prepared with the mixed carrier material was both higher than those of the corresponding Composition 3-1 and 3-2 prepared with single carrier material. This indicated that use of mixed carrier materials could significantly improve the dissolution of Olaparib, and further improve the absorption thereof *in vivo,* compared to the use of single carrier material.
2) The dissolution of pharmaceutical Composition 3-2 and Composition 3-4 containing TPGS was higher than that of Composition 3-1 and 3-3 without TPGS, indicating that the solubilizer could further improve the dissolution of Olaparib, and could also further improve the absorption of Olaparib *in vivo.*

### Example 4 Evaluation of the preparation processes of the mixed carrier pharmaceutical composition

### 1. Hot melt extrusion process

**Table 4-1 Hot melt extrusion process parameters**

| Compositions | Feeder speed (rpm) | Screw speed (rpm) | Material melting temperature (°C) | Energy consumption per kilogram (kW/h) | Torque percentage (%) |
|---|---|---|---|---|---|
| 1-3 | 35 | 160 | 142 | 8 | 46 |
| 2-1 | 45 | 200 | 154 | 10 | 52 |
| 2-2 | 40 | 180 | 139 | 5 | 38 |
| 2-3 | 40 | 180 | 140 | 4 | 32 |
| 2-4 | 40 | 180 | 138 | 4 | 31 |

The processes were shown in the preparation of each pharmaceutical composition in Example 1-3, and the specific parameters were shown in Table 4-1.

As shown in Table 4-1, compared with Composition 2-1 prepared with single second carrier material (Kollidon^{®} VA64), Composition 1-3 prepared with single cyclodextrin carrier material (Kleptose) had lower energy consumption per kilogram and lower percent torque produced.

The glass transition temperatures of Composition 2-2, Composition 2-3 and Composition 2-4 prepared with mixed carrier materials were all higher than those of Composition 2-1 prepared with single carrier material, and the Tg value was significantly different from Olaparib, indicating that Olaparib and the carrier material formed a glass solution, the viscosity of the component mixture of composition using the mixed carrier was relatively lower, and the screw speed during the process was reduced. Correspondingly, Compositions 2-2, 2-3 and 2-4 prepared with mixed carrier materials had lower energy consumption per kilogram and lower torque percentages produced. It indicated that the use of mixed carrier materials to prepare pharmaceutical compositions could significantly reduce the energy consumption and instrument torque of the hot melt extrusion process, and greatly improved operability thereof.

### 2. Pulverization process

**Table 4-2 Process parameters of extrudate pulverization**

| Compositions | Extrudate weight (g) | Pulverization time (s) | Pulverization speed (rpm) | Weight of particles passing through a 60-mesh sieve (g) | Pulverization yield (%) |
|---|---|---|---|---|---|
| 1-3 | 162.3 | 60 | 4000 | 127.1 | 78.3 |
| 2-1 | 167.2 | 60 | 4000 | 67.7 | 40.5 |
| 2-2 | 168.5 | 60 | 4000 | 113.7 | 67.5 |
| 2-3 | 170.2 | 60 | 4000 | 116.1 | 68.2 |
| 2-4 | 175.4 | 60 | 4000 | 123.0 | 70.1 |

The extrudates prepared by each pharmaceutical composition were cut into strips of about 2 cm with scissors, and pulverized with a Fitzmill pulverizer (model: RP-L1A) at 4000 rpm for 60 s. The pulverized particles were passed through a 60-mesh sieve, the weight of the powder obtained after drying was weighed, and the pulverization yield was calculated.

As shown in Table 4-2, compared with the sieving efficiency (40.5%) of Composition 2-1 prepared with single second carrier material (Kollidon^{®} VA64), the sieving efficiency of the pharmaceutical composition prepared with mixed carrier materials or single cyclodextrin carrier material was higher (higher than 67%), indicating that the pulverization process was more operable.

### Example 5 In vivo pharmacokinetic study of Olaparib-mixed carrier pharmaceutical composition

An open-label, randomized, two-period crossover, self-controlled, comparative pharmacokinetics study was conducted in human subjects under fasting condition.

The included 10 healthy subjects were randomly divided into 2 groups: Group 1, Group 2, 5 people in each group. Group 1 first orally took the tablet containing Composition 2-1 prepared in Example 5, and Group 2 first orally took the tablet containing Composition 2-3 prepared in Example 5, and the cross-experiment was conducted after a 1-week washout period, and the grouping scheme was shown in the following table:

**Table 5-1 Grouping**

| Period | Group 1 | Group 2 |
|---|---|---|
| Period I | Composition 2-1 | Composition 2-3 |
| Period II | Composition 2-3 | Composition 2-1 |

At 0 h, 0.25 h, 0.5 h, 1.0 h, 1.5 h, 2.0 h, 2.5 h, 3.0 h, 3.5 h, 4.0 h, 5.0 h, 7.0 h, 9.0 h, 12.0 h, 24.0 h, 48.0 h after administration (total 16 blood collection points), 4 mL of cubital venous blood was collected, respectively, then centrifuged, extracted and separated for plasma, and stored in -20°C refrigerator for testing.

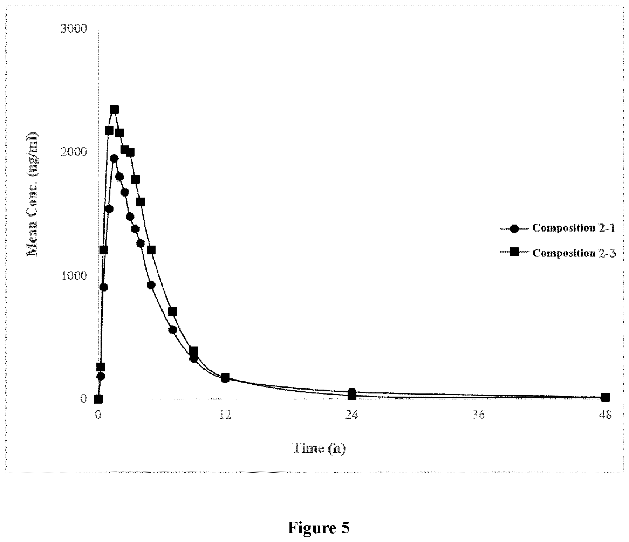

The concentration of Olaparib in each plasma sample was determined by LC-MS/MS method and calculated by the pharmacokinetic statistical software WinNonlin v6.4 for the biostatistical analysis. The test results were shown in Table 5-2 and Figure 5.

**Table 5-2 Geometric mean statistics of Olaparib pharmacokinetic parameters (n = 10)**

| Pharmacokinetic parameters (Geometric mean) | Composition 2-1 | Composition 2-3 | Geometric mean ratio |
|---|---|---|---|
| | | | Composition 2-3 / Composition 2-1 |
| LnCₘₐₓ (ng·ml⁻¹) | 2114.51 | 2694.84 | 1.27 |
| LnAUC₀₋₄₈ₕ (ng·h·ml⁻¹) | 10781.88 | 13459.44 | 1.25 |
| LnAUC_{0-∞} (ng·h·ml⁻¹) | 11037.67 | 13585.67 | 1.23 |

As can be seen from Figure 5 and Table 5-1, compared with Composition 2-1 using single second carrier material (Kollidon^{®} VA64), the absorption of Olaparib in Composition 2-3 using mixed carrier material (Kleptose/Kollidon^{®} VA64) was faster *in vivo,* with higher plasma concentration and corresponding higher bioavailability. The geometric mean ratio of Cₘₐₓ (Composition 2-3/Composition 2-1) was 1.27; the geometric mean ratios of AUC₀₋₄₈ₕ and AUC_{0-∞} (Composition 2-3/Composition 2-1) were 1.25 and 1.23, respectively.

In summary, the Olaparib composition prepared with mixed carrier material had significantly improved *in vitro* solubility and dissolution compared with the Olaparib composition prepared with single carrier material, and the *in vivo* Cₘₐₓ and bioavailability were also improved as a whole.

Those skilled in the art will understand that the above contents of the disclosure are further described in detail through the specific embodiments in the form of examples. But it should not be construed that the scope of the subject matter of the present disclosure is limited to the above examples. The solutions implemented based on the above contents of the present disclosure are all within the scope of the present disclosure.

## Claims

1. A pharmaceutical composition, comprising an active ingredient Olaparib and a carrier material cyclodextrin, wherein
the cyclodextrin is one or more selected from the group consisting of α cyclodextrin, γ cyclodextrin, β cyclodextrin, hydroxypropyl β cyclodextrin, hydroxypropyl α cyclodextrin, sulfobutyl β cyclodextrin, and methyl β cyclodextrin.

2. The pharmaceutical composition according to claim 1, wherein
the carrier material cyclodextrin is hydroxypropyl β cyclodextrin.

3. The pharmaceutical composition according to claim 1 or 2, wherein
the weight ratio of the active ingredient Olaparib to the carrier material cyclodextrin is about 1:0.5 - about 1:5, preferably about 1:0.5 - about 1:4, more preferably about 1:0.5 - about 1:3.

4. The pharmaceutical composition according to any one of claims 1-3, wherein
in addition to the carrier material cyclodextrin, the pharmaceutical composition further comprises a second carrier material,
preferably, the second carrier material is one or more selected from the group consisting of copovidone, povidone, polyvinyl caprolactam-polyvinyl acetate-polyethylene glycol graft copolymer, polyoxyethylene, hypromellose and derivatives thereof, hydroxypropyl cellulose, methyl cellulose, hydroxyethyl cellulose, ethyl cellulose, sodium alginate, acrylic resin, and carbomer;
more preferably, the second carrier material is one or more selected from the group consisting of copovidone, polyethylene glycol/vinyl caprolactam/vinyl acetate copolymer, acrylic resin, hypromellose and derivatives thereof;
particularly preferably, the second carrier material is one or more selected from the group consisting of copovidone, and polyethylene glycol/vinyl caprolactam/vinyl acetate copolymer.

5. The pharmaceutical composition according to claim 4, wherein
the weight ratio of the active ingredient Olaparib to the second carrier material is about 1:0.5 - about 1:5, preferably about 1:0.5 - about 1:3, more preferably about 1:0.5 - about 1:2.5.

6. The pharmaceutical composition according to claim 4 or 5, wherein
the weight ratio of the carrier material cyclodextrin to the second carrier material is about 1:1 - about 1:10, preferably about 1:1 - about 1:8, more preferably about 1:1 - about 1:3.

7. The pharmaceutical composition according to any one of claims 1-6, wherein
the weight ratio of the active ingredient Olaparib to the total weight of the carrier material cyclodextrin and the second carrier is about 1:0.5 - about 1:5, preferably about 1:0.5 - about 1:4, more preferably about 1:0.5 - about 1:3.

8. The pharmaceutical composition according to any one of claims 1-7, wherein
the pharmaceutical composition further comprises a solubilizer,
preferably, the solubilizer is polyethylene glycol 1000 vitamin E succinate (TPGS), more preferably, the weight ratio of the solubilizer to the total weight of the active ingredient Olaparib, the carrier material cyclodextrin and/or the second carrier material and the solubilizer is about 0.5-12%, preferably about 7%-11%.

9. The pharmaceutical composition according to any one of claims 1-8, wherein
the active ingredient Olaparib is dissolved in the carrier material cyclodextrin and/or the second carrier material or dispersed at the molecular level in the carrier material cyclodextrin and/or the second carrier material.

10. A solid pharmaceutical formulation comprising the pharmaceutical composition according to any one of claims 1-9;
preferably, the pharmaceutical formulation is in the form of a powder, a granule, a pill, a capsule or a tablet.

11. The solid pharmaceutical formulation according to claim 10, wherein
the pharmaceutical formulation further comprises a pharmaceutically acceptable excipient,
the excipient is preferably one or more of glidant, diluent, disintegrant, binder and lubricant.

12. Use of the pharmaceutical composition according to any one of claims 1-9 or the pharmaceutical formulation of claim 10 or 11 in the manufacture of a medicament for the treatment of a malignant tumor or related disease,
preferably, the malignant tumor or related disease is an ovarian cancer, a breast cancer, a pancreatic cancer, a prostate cancer, a non-small cell lung cancer, an esophageal cancer or a gastric cancer.

13. A process for preparing the pharmaceutical composition according to any one of claims 1-9, comprising:
1) providing the active ingredient Olaparib and carrier material;
2) adding the materials of step 1) into a hot melt extruder and performing extruding at about 120°C-190°C;
3) cooling, pulverizing and sieving the extrudate obtained in step 2) to obtain the pharmaceutical composition.
